(19) Europäisches Patentamt
European Patent Office
Office européen des brevets

(11) EP 2 117 652 B1

(12) **EUROPEAN PATENT SPECIFICATION**

(45) Date of publication and mention of the grant of the patent:
**25.09.2013 Bulletin 2013/39**

(21) Application number: **08716097.4**

(22) Date of filing: **28.02.2008**

(51) Int Cl.:
***A61Q 19/02*** *(2006.01)* ***A61Q 19/08*** *(2006.01)*
***A61K 8/49*** *(2006.01)* ***A61K 8/67*** *(2006.01)*
***A61P 17/10*** *(2006.01)*

(86) International application number:
**PCT/EP2008/001568**

(87) International publication number:
**WO 2008/110268 (18.09.2008 Gazette 2008/38)**

(54) **COSMETIC COMPOSITIONS**

KOSMETISCHE ZUSAMMENSETZUNGEN

COMPOSITIONS COSMÉTIQUES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **12.03.2007 EP 07005028**

(43) Date of publication of application:
**18.11.2009 Bulletin 2009/47**

(73) Proprietor: **DSM IP Assets B.V.**
**6411 TE Heerlen (NL)**

(72) Inventors:
- **Den-Brave, Kerstin**
  **CH-4058 Basel (CH)**
- **Saecker, Christine**
  **CH-5023 Biberstein (CH)**
- **Westenfelder, Horst**
  **67435 Neustadt a.d.W. (DE)**

(74) Representative: **Schwander, Kuno et al**
**DSM Nutritional Products Ltd**
**Patent Department**
**Wurmisweg 576**
**4303 Kaiseraugst (CH)**

(56) References cited:
**WO-A-02/11675** **WO-A-99/49878**
**WO-A-2004/062635** **WO-A-2005/041996**
**WO-A-2005/092283** **DE-A1- 19 509 354**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 117 652 B1

## Description

The present invention relates to the use of ascorbyl phosphate and niacinamide to suppress the crystallization of biotin in cosmetic or dermatological compositions. Furthermore, the invention relates to cosmetic or dermatological compositions comprising ascorbyl phosphate, niacinamide, biotin and a cosmetically acceptable carrier.

Biotin is used in cosmetic or dermatological compositions e.g. as skin-conditioning agent, skin lightening agent or anti-ageing agent. However, biotin is not very soluble (about 0.02g /100ml of hot water). Thus, even if used in low amounts biotin tends to crystallize in cosmetic or dermatological compositions leading to a loss of activity as well as to an aesthetically unpleasant appearance of the composition. Because of these reason, up to today biotin may only be used in very low concentration in cosmetic or dermatological compositions. Thus, there is a need for agents which enhance the solubility of biotin and consequently reduce the crystallization of biotin in cosmetic or dermatological compositions.

Ascorbyl phosphates are used in skin care products in particular as in-vivo antioxidants, as skin lightening or anti acne agents. However, ascorbyl phosphates incorporated in higher concentrations into cosmetic and dermatological compositions often lead to an unpleasant brown discoloration of the products, which is not well accepted by the end consumer. Thus, there is an ongoing need of reducing the amount of ascorbyl phosphate incorporated into cosmetic or dermatological compositions while maintaining the same efficiency of the products in respect of the antioxidant, skin lightening or anti acne activity by the addition of further ingredients such as biotin.

Surprisingly, it has been found that a combination of ascorbyl phosphate and niacinamide significantly enhances the solubility of biotin in water which, in consequence, allows the incorporation of more biotin into cosmetic or dermatological compositions. Furthermore, it has been found, that the combination of ascorbyl phosphate and niacinamide reduces the re- crystallization of biotin in cosmetic or dermatological compositions while exhibiting a negligible discoloration and providing excellent cosmetic benefits such as moisturizing, anti- aging, anti- acne or skin- lightening.

Thus, in one aspect, the invention relates to cosmetic or dermatological compositions comprising an ascorbyl phosphate, niacinamide, biotin and a cosmetically acceptable carrier.

In a further aspect, the present invention is related to cosmetic or dermatological compositions comprising

(a) 0.1 to 5 wt. %, preferably 0.3 to 3 wt. %, most preferably 0.3 to 1% of an ascorbyl phosphate;
(b) 0.5 to 10 wt. % , preferably 1 to 10 wt. %, most preferably 1 to 5 wt. % of niacinamide
(c) 0.005 to 3 wt.-% of biotin, preferably 0.01 to 1 wt.-%, most preferably 0.05 to 0.5 wt.% and
(d) a cosmetically acceptable carrier.

Such cosmetic compositions show excellent cosmetic benefits e.g. in regard of moisturizing, anti-aging, anti-acne or skin-lightening while exhibiting a negligible discoloration.

If nothing else is stated, in this specification parts and percentages are per weight and are based on the total weight of the composition.

In a preferred embodiment the invention relates to cosmetic or dermatological compositions according to the invention furthermore comprising 20 to 99 wt.-%, preferably 30 to 90 wt.-%, most preferably 40 to 80 wt. % of water.

In all embodiments of the invention preferably the amount of ascorbyl phosphate as well as the amount of niacinamide is higher than the amount of biotin used. Advantageously, the amount of ascorbyl phosphate as well as the amount of niacinamide is at least 1.5 times the amount of biotin used.

In all embodiments of the invention preferably the amount of niacinamide is equal or higher than the amount of ascorbyl phosphate used. Preferably, the ratio of niacinamide to the ascorbyl phosphate is selected in the range of about 1:1 to 1:100, preferably in the range of about 1:1 to 1:50, in particular in the range of about 1:1 to 1:5 such as in a ratio of about 1:3.

The term biotin as used herein refers to a compound of formula (1)

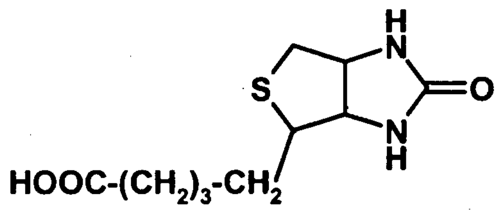

(1)

which encompasses 8 different stereoisomers. In all embodiments of the invention, the biotin can either be used in a stereochemically pure form or as a mixture of two or several stereoisomers. Preferably, D- (+)- Biotin, namely (3aS, 4S,6aR)- 2- Oxohexahydrothieno [3, 4- d]- imidazol- 4- valerianic acid of formula (2)

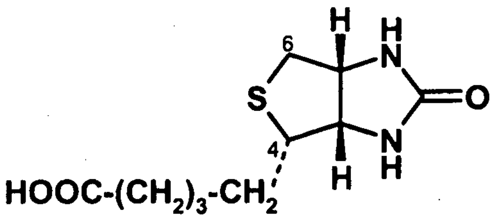

(2)

is used in all embodiments of the invention.

The term biotin as used in the context of this invention also encompasses biotin in the form of a salt. Suitable salts are the salts of biotin with alkali or earth alkaline metals or other suitable metals such as sodium, potassium or magnesium salts, but also ammonium salts. Because of the nitrogen atoms, biotin can also be used in the form of a salt generated by addition of an inorganic or organic acid such as HCl to the nitrogen atom(s) of biotin. The use of biotin hydrochloride is particularly preferred. The preparation of the salts can be performed by methods known to a person skilled in the art, e.g. by reacting biotin with a base such as NaOH or KOH or an acid such as HCl.

The term "ascorbyl phosphate" as used herein denotes metal salts of mono- and polyphosphoric acid esters of ascorbic acid wherein the phosphorylated hydroxy group of the ascorbic acid molecule features one or more phosphoric acid (phosphate) units, and metal cations, e.g. sodium and/or magnesium or calcium ions, are also present. The term "poly" generally denotes 2- 10, preferably 2- 4, phosphate units. The ascorbyl phosphates may also be referred to in general as "ascorbyl (poly) phosphates" to embrace both mono- and polyphosphates. Typical ascorbyl phosphates for use in the present invention are L- ascorbic acid phosphate ester salts such as sodium ascorbyl phosphate, potassium ascorbyl phosphate, magnesium ascorbyl phosphate, calcium ascorbyl phosphate and sodium magnesium L- ascorbyl-2- monophosphate. The ascorbyl phosphates are essentially present in the form of a hydrate or a dihydrate. Commercially available ascorbyl phosphates comprise trisodium L- ascorbyl- 2- monophosphate which is available as STAY- C®50 from DSM Nutritional Products AG, (4303 Kaiseraugst, Switzerland) and magnesium L- ascorbyl phosphate (available from Showa Denko) and sodium magnesium L- ascorbyl- 2- monophosphate. The preferred ascorbyl phosphate for the purposes of the present invention are sodium or sodium magnesium or sodium calcium ascorbyl phosphate or mixtures thereof, in particular trisodium L- ascorbyl- 2- monophosphate dihydrate.

Niacinamide [CAS-Nr. 98-92-0] is one of the water-soluble B-complex vitamins which is e.g. available as Niacinamide PC or Niacinamide from DSM Nutritional Products AG, (4303 Kaiseraugst, Switzerland). Niacinamide is also referred to as nicotinamide or pyridine-3-carboxamide and is the amide of niacin (vitamin $B_3$).

Preferred are cosmetic or dermatological compositions as defined above which are topical compositions e.g. skin-conditioning and moisturizing compositions, skin-lightening compositions and age spot treatments, anti-ageing or regenerating compositions or compositions for the prevention, reduction or treatment of wrinkles and antioxidant or free radical scavenging composition and purifying, cleansing, exfoliating or deodorizing compositions, anti-acne or anti-skin blemishes compositions.

The present invention also pertains to the use of the cosmetic or dermatological compositions as defined above for beautifying and hydrating the skin including those to sooth dry and irritated skin and to strengthen the skin's natural defense, for skin-lightening and age spot treatment or for the prevention, reduction or treatment of skin ageing and/ or wrinkles or to stimulate cell renewal and a firmer, radiant looking skin. The composition can be also used to prevent and combat blemishes of acne skin and to purify, cleanse, exfoliate or deodorize the skin and to refine the complexion. Preferably, the invention relates to the use of the cosmetic or dermatological compositions according to the invention as moisturizing, skin-lightening agent, or for the prevention, reduction or treatment of wrinkles. Also, the invention pertains to a composition for use as an anti-aging or anti-acne agent.

Furthermore the invention relates to a method of lightening the skin, a method of providing an anti-ageing benefit and/or a method of prevention, reduction or treatment of wrinkles, in particular a method of lightening the skin the method comprising the step of applying an effective amount of a cosmetic or dermatological composition as defined above to the skin of a subject in need of such a treatment. Furthermore, the invention relates to a method of preventing and reducing skin blemishes associated with acne the method comprising the step of applying an effective amount of a cosmetic or dermatological composition as defined above to the skin of a subject in need of such a treatment.

The term skin lightening encompasses an elucidation of the natural skin tone of a person which e.g. because of cultural reasons like to have a lighter taint as well as to the minimization or even elimination of skin hyper-pigmentation and/or pigmentation disorders such as age spots, freckles, blotches, darkening, uneven tone.

The term preventing and reducing skin blemishes associated with acne encompasses prevention and treatment of black and white spots, inflammatory and non-inflammatory skin lesions, oily and unevenly looking skin tone.

The topical application is preferably applied at least once per day but can also be applied several times a day

e.g. two or three times a day. Usually it takes at least two days until the desired effect is achieved. However, it can take several weeks or even months until the desired effect is achieved.

The amount of the cosmetic or dermatological composition which is to be applied to the skin depends on the concentration of the active ingredients in the compositions and the desired cosmetic or pharmaceutical effect. For example, application can be such that a crème is applied to the skin. A cream is usually applied in an amount of about 1 to 2 mg crème/$cm^2$ skin. The amount of the composition which is applied to the skin is, however, not critical, and if with a certain amount of applied composition the desired effect cannot be achieved, a higher concentration of the active ingredients can be used e.g. by applying more of the composition or by applying compositions which contain more active ingredient.

The cosmetic or dermatological compositions according to the invention may be in the form of a suspension or dispersion in solvents or fatty substances, or alternatively in the form of an emulsion or micro emulsion (in particular of O/W or W/O type, O/W/O or W/O/W-type, wherein O stands for organic phase and wherein W stands for water phase), such as a cream, a paste, a lotion, a thickened lotion or a milk, a vesicular dispersion in the form of an ointment, a gel, a solid tube stick or an aerosol mousse, and may be provided in the form of a mousse, foam or a spray foams, sprays, sticks or aerosols or wipes. Examples of cosmetic or dermatological compositions according to the invention are skin care preparations, in particular, body lotions, body creams, body foams, body gels, facial lotions, facial creams, facial gels, e.g. eye creams, anti-wrinkle creams, day care lotions, night creams, treatment creams, treatment solutions, skin protection ointments, sunscreens, moisturizing gels, moisturizing creams, moisturizing sprays, revitalizing body sprays, anti-cellulite gels/creams, anti-acne preparations, cleansing milks/gels and peeling/exfoliating preparations.

The cosmetic or dermatological compositions according to the invention have a pH in the range of 3-10, preferably in the range of pH of 4-8, most preferred in the range of pH 5.5-7.5.

The term cosmetically acceptable carrier refers to all vehicles/ carriers conventionally used in cosmetic compositions.

The cosmetic or dermatological compositions according to the invention such as the skin care preparations can contain further adjuvants and additives such as preservatives/ antioxidants, fatty substances/ oils, water, organic solvents, silicones, thickeners, softeners, emulsifiers, light screening agents, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nano-pigments, active ingredients, preservatives, insect repellants, or any other ingredients usually formulated into cosmetics. The necessary amounts of the cosmetic and dermatological adjuvants and additives can, based on the desired product, easily be chosen by a skilled artisan in this field and will be illustrated in the examples, without being limited hereto.

<u>Light screening agents</u>

Additional screening agents which can be incorporated into the cosmetic or dermatological compositions according to the invention are advantageously selected from IR, UV- A, UV- B, UV- C and/ or broadband filters. Examples of UV- B or broad spectrum screening agents, i.e. substances having absorption maximums between about 290 nm and 340 nm may be organic or inorganic compounds. Organic UV- B or broadband screening agents are e.g. acrylates such as 2- ethylhexyl 2- cyano- 3, 3- diphenylacrylate (octocrylene, PARSOL® 340), ethyl 2- cyano- 3, 3- diphenylacrylate; camphor derivatives such as 4- methyl benzylidene camphor (PARSOL® 5000), 3- benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid; Cinnamate derivatives such as ethylhexyl methoxycinnamate (PARSOL® MCX), ethoxyethyl methoxycinnamate, diethanolamine methoxycinnamate (PARSOL® Hydro), isoamyl methoxycinnamate as well as cinnamic acid derivatives bond to siloxanes; p- aminobenzoic acid derivatives, such as p- aminobenzoic acid, 2- ethylhexyl p- dimethylaminobenzoate, N- oxypropylenated ethyl p- aminobenzoate, glyceryl p- aminobenzoate; benzophenones such as benzophenone- 3, benzophenone- 4, 2, 2', 4, 4'- tetrahydroxybenzophenone, 2, 2'- dihydroxy- 4, 4'- dimethoxybenzophenone, esters of benzalmalonic acid such as di- (2- ethylhexyl) 4- methoxybenzalmalonate; esters of 2- (4- ethoxy- anilinomethylene) propandioic acid such as 2- (4- ethoxy anilinomethylene) propandioic acid diethyl ester as described in the European Patent Publication EP 0895 776; organosiloxane compounds containing benzmalonate groups as described in the European Patent Publications EP 0358584 B1, EP 0538431 B1 and EP 0709080 A1 such as PARSOL® SLX; drometrizole trisiloxane (Mexoryl XL) ; imidazole derivatives such as e.g. 2- phenyl benzimidazole sulfonic acid and its salts (PARSOL®HS) . Salts of 2- phenyl benzimidazole sulfonic acid are e.g. alkali salts such as sodium- or potassium salts, ammonium salts, morpholine salts, salts of primary, sec. and tert. amines like monoethanolamine salts, diethanolamine salts; salicylate derivatives such as isopropylbenzyl salicylate, benzyl salicylate, butyl salicylate, ethylhexyl salicylate (PARSOL® EHS, Neo Heliopan OS), isooctyl salicylate or homomenthyl salicylate (homosalate, PARSOL® HMS, Neo Heliopan HMS) ; triazine derivatives such as ethylhexyl triazone (Uvinul T- 150), diethylhexyl butamido triazone (Uvasorb HEB) . Encapsulated UV- filters such as encapsulated ethylhexyl methoxycinnamate (Eusolex UV- pearls) or microcapsules loaded with UV- filters as e.g. dislosed in EP

1471995; Inorganic compounds are pigments such as microparticulated $TiO_2$. The term "microparticulated" refers to a particle size from about 5 nm to about 200 nm, particularly from about 15 nm to about 100 nm. The $TiO_2$ particles may also be coated by metal oxides such as e.g. aluminum or zirconium oxides or by organic coatings such as e.g. polyols, methicone, aluminum stearate, alkyl silane. Such coatings are well known in the art. Commercially available titanium dioxide e.g. encompass PARSOL TX obtainable at DSM Nutritional Products without being limited thereto.

Examples of broad spectrum or UV A screening agents i.e. substances having absorption maximums between about 320 nm and 400 nm may be organic or inorganic compounds e.g. dibenzoylmethane derivatives such as 4- tert.-butyl- 4'- methoxydibenzoyl- methane (PARSOL® 1789), dimethoxydibenzoylmethane, isopropyldibenzoylmethane; benzotriazole derivatives such as 2, 2'- methylene- bis- (6- (2H- benzotriazole- 2- yl)- 4- (1, 1, 3, 3, -tetramethylbutyl)- phenol (Tinosorb M) ; bis- ethylhexyloxyphenol methoxyphenyl triazine (Tinosorb S) ; phenylene- 1, 4- bis- benzimidazolsulfonic acids or salts such as 2, 2- (1, 4- phenylene) bis- (1H- benzimidazol- 4, 6- disulfonic acid) (Neoheliopan AP) ; amino substituted hydroxybenzophenones such as 2- (4- Diethylamino- 2- hydroxy- benzoyl)- benzoic acid hexylester (Uvinul A plus) as described in the European Patent Publication EP 1046391; Ionic UV- A filters as described in the International Patent Publication WO2005080341 A1; Pigments such as microparticulated ZnO or $TiO_2$. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art.

As dibenzoylmethane derivatives have limited photostability it may be desirable to photostabilize these UV-A screening agents. Thus, the term "conventional UV-A screening agent" also refers to dibenzoylmethane derivatives such as e.g. PARSOL® 1789 stabilized by, e.g. 3,3-Diphenylacrylate derivatives as described in the European Patent Publications EP 0 514 491 B1 and EP 0 780 119 A1; Benzylidene camphor derivatives as described in the US Patent No. 5,605,680; Organosiloxanes containing benzmalonate groups as described in the European Patent Publications EP 0358584 B1, EP 0538431 B1 and EP 0709080 A1.

Antioxidants

Based on the invention all known antioxidants usually formulated into body care and household products can be used. Especially preferred are antioxidants chosen from the group consisting of amino acids (e.g. glycine, histidine, tyrosine, tryptophan) and their derivatives, imidazole (e.g. urocanic acid) and derivatives, peptides such as D, L- carnosine, D- carnosine, L- carnosine and derivatives (e.g. anserine), carotenoids, carotenes (e.g. $\alpha$- carotene, $\beta$- carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cysteine, cystine, cystamine and its glycosyl-, N- acetyl-, methyl-, ethyl-, propyl-, amyl-, butyl- and lauryl-, palmitoyl- ; oleyl-, linoleyl-, cholesteryl- and glycerylester) and the salts thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinesulfoximine, buthioninsulfone, penta-, hexa-, heptathioninsulfoximine) in very low compatible doses (e.g. pmol bis $\mu$mol/ kg), additionally (metal)- chelators (such as $\alpha$- hydroxyfatty acids, palmic-, phytinic acid, lactoferrin), $\beta$- hydroxyacids (such as citric acid, lactic acid, malic acid), huminic acid, gallic acid, gallic extracts, bilirubin, biliverdin, EDTA, EGTA and its derivatives, unsaturated fatty acids and their derivatives (such as $\gamma$- linoleic acid, linolic acid, oleic acid), folic acid and its derivatives, ubiquinone and ubiquinol and their derivatives, vitamin C and derivatives (such as ascorbylpalmitate and ascorbyltetraisopalmitate, ascorbyl- acetate, ascorbyl glucoside), tocopherol and derivates (such as vitamin- E- acetate), mixtures of nat. vitamin E, vitamin A and derivatives (vitamin- A- palmitate and- acetate) as well as coniferylbenzoate, rutinic acid and derivatives, $\alpha$- glycosylrutin, ferulic acid, furfurylideneglucitol, carnosine, butylhydroxytoluene, butylhydroxyanisole, trihydroxybutyrophenone, urea and its derivatives, mannose and derivatives, zinc and derivatives (e.g. ZnO, $ZnSO_4$) selen and derivatives (e.g. selenomethionin), stilbenes and derivatives (such as stilbenoxide, trans- stilbenoxide) and suitable derivatives (salts, esters, ethers, sugars, nucleotides, nucleosides, peptides and lipids) of the named active ingredients. One or more preservatives/ antioxidants may be present in an amount of at least 0.01 wt. % of the total weight of the composition. Preferably about 0.01 wt. % to about 10 wt. % of the total weight of the composition of the present invention is present. Most preferred, one or more preservatives/ antioxidants are present in an amount about 0.1 wt. % to about 1 wt. %.

Surface active ingredients

Typically cosmetic or dermatological compositions also contain surface active ingredients like emulsifiers, solubilizers. An emulsifier enables two or more immiscible components to be combined homogeneously. Moreover, the emulsifier acts to stabilize the composition. Emulsifiers that may be used in the present invention in order to form O/W, W/O, O/W/O or W/O/W emulsions/ micro emulsions include sorbitan oleate, sorbitan sesquioleate, sorbitan isostearate, sorbitan trioleate, polyglyceryl- 3- diisostearate, polyglycerol esters of oleic/ isostearic acid, polyglyceryl- 6 hexaricinolate,

polyglyceryl- 4- oleate, polygylceryl- 4 oleate/ PEG- 8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further exemplary emulsifiers are phosphate esters and the salts thereof such as cetyl phosphate (Amphisol® A), diethanolamine cetyl phosphate (Amphisol® DEA), potassium cetyl phosphate (Amphisol® K), sodium glyceryl oleate phosphate, hydrogenated vegetable glycerides phosphate and mixtures thereof. Furthermore, one or more synthetic polymers may be used as an emulsifier. For example, PVP eicosene copolymer, acrylates/ $C_{10-30}$ alkyl acrylate crosspolymer, acrylates/ steareth- 20 methacrylate copolymer, PEG-22/ dodecyl glycol copolymer, PEG- 45/ dodecyl glycol copolymer, and mixtures thereof. Further exemplary emulsifiers are fatty alcohols, e.g. cetearyl alcohol (Lanette O, Cognis Coopearation), cetyl alcohol (Lanette 16, Cognis Cooperation), stearyl alcohol (Lanette 18, Cognis Cooperation), Laneth- 5 (Polychol 5, Croda Chemicals), furthermore sucrose and glucose derivatives, e.g. sucrose distearate (Crodesta F- 10, Croda Chemicals), Methyl glucose isostearate (Isolan IS, Degussa Care Chemicals), furthermore ethoxylated carboxylic acids or polyethyleneglycol esters and polyethyleneglycol ethers, e.g. steareth- 2 (Brij 72, Uniqema), steareth- 21 (Brij 721, Uniqema), ceteareth- 25 (Cremophor A25, BASF Cooperation), PEG- 40 hydrogenated castor oil (Cremophor RH- 40, BASF Cooperation), PEG- 7 hydrogenated castor oil (Cremophor WO7, BASF Cooperation), PEG- 30 Dipolyhydroxystearate (Arlacel P 135, Uniqema), furthermore glyceryl esters and polyglyceryl esters, e.g. polyglyceryl- 3- diisostearate (Hostacerin TGI, Clariant Cooperation), polyglyceryl-2 dipolyhydroxystearate (Dehymuls PGPH, Cognis Cooperation), polyglyceryl- 3 methylglucose distearate (Tego Care 450, Degussa Care Chemicals) . The preferred emulsifiers are cetyl phosphate (Amphisol® A), diethanolamine cetyl phosphate (Amphisol® DEA), potassium cetyl phosphate (Amphisol® K), PVP Eicosene copolymer, acrylates/ $C_{10-30}$-alkyl acrylate crosspolymer, PEG- 20 sorbitan isostearate, sorbitan isostearate, and mixtures thereof. The one or more emulsifiers are present in a total amount of at least 0.01 wt. % of the total weight of the composition. Preferably about 0.01 wt. % to about 20 wt. % of the total weight of the composition of the present invention is used. Most preferred, about 0.1 wt. % to about 10 wt. % of emulsifiers are used.

Typically cosmetic or dermatological compositions may also contain anionic, neutral, amphoteric or cationic tensides. Exemplary anionic tensides comprise alkylsulfate, alkylethersulfate, alkylsulfonate, alkylarylsulfonate, alkylsuccinate, alkylsulfosuccinate, N-alkoylsarkosinate, acyltaurate, acylisethionate, alkylphosphate, alkyletherphosphate, alkylethercarboxylate, alpha-olefinsulfonate, especially the alkali-und earth alkali salts, e.g. sodium, potassium, magnesium, calcium, as well as ammonium- and triethanol amine-salts. The alkylethersulfate, alkyletherphosphate and alkylethercarboxylate may comprise between 1 to 10 ethylenoxide or propylenoxide units, preferably 1 to 3 ethylenoxide-units per molecule.

Suitable are e.g. sodium laurylsulfate, ammonium lauryl sulfate, sodium laurylethersulfate, ammonium laurylethersulfate, sodium lauroylsarkonisate, sodiumoleylsuccinate, ammonium laurylsulfosuccinate, sodium dodecylbenzolsulfonate, triethanolamidodecylbenzolsulfonate.

Suitable amphoteric tensides are e.g. alkylbetaine, alkylamidopropylbetaine, alkylsulfobetaine, alkylglycinate, alkylcarboxyglycinate, alkylamphoacetate or propionate, alkylamphodiacetate or dipropionate such as cocodimethylsulfopropylbetain, laurylbetain, cocamidopropylbetain or sodium cocamphopropionate.

Furthermore, the cosmetic or dermatological compositions may contain the usual cationic tensides such as quaternized ammonium compounds e.g. cetyltrimethylammoniumchlorid or bromide (INCI: cetrimoniumchloride or bromide), hydroxyethylcetyldimonium phosphate (INCI: Quaternium- 44), Luviquat® Mono LS (INCI: Cocotrimoniummethosulfate), poly (oxy- 1, 2- Ethandiyl), (Octadecylnitrilio) tri- 2, 1- Ethandiyl) tris- (hydroxy)- phosphate (INCI Quaternium-52) .

The one or more anionic, neutral, amphoteric or cationic tensides are present in a total amount of at least 0.01wt. % of the total weight of the composition. Preferably about 0.01 wt. % to about 20 wt. % of the total weight of the composition of the present invention is used. Most preferred, about 0.1 wt. % to about 10 wt. % of one or more tensides are used.

Oil and fatty components

The lipid phase of cosmetic or dermatological compositions can advantageously be chosen from mineral oils and mineral waxes; oils such as triglycerides of caprinic acid and/ or caprylic acid or castor oil; oils or waxes and other natural or synthetic oils, in an preferred embodiment esters of fatty acids with alcohols e.g. isopropanol, propyleneglycol, glycerin or esters of fatty alcohols with carbonic acids or fatty acids; alkylbenzoates; and/ or silicone oils.

Exemplary fatty substances which can be incorporated in the oil phase of the emulsion, micro emulsion, oleo gel, hydrodispersion or lipodispersion of the present invention are advantageously chosen from esters of saturated and/ or unsaturated, linear or branched alkyl carboxylic acids with 3 to 30 carbon atoms, and saturated and/ or unsaturated, linear and/ or branched alcohols with 3 to 30 carbon atoms as well as esters of aromatic carboxylic acids and of saturated and/ or unsaturated, linear or branched alcohols of 3-30 carbon atoms. Such esters can advantageously be selected from octylpalmitate, octylcocoate, octylisostearate, octyldodecylmyristate, cetearylisononanoate, isopropylmyristate, isopropylpalmitate, isopropylstearate, isopropyloleate, n-butylstearate, n-hexyllaurate, n-decyloleate, isooctylstearate, isononylstearate, isononylisononanoate, 2-ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as synthetic, half-synthetic or natural mixtures of such esters e.g. jojoba oil.

Other fatty components suitable for cosmetic or dermatological compositions of the present invention include polar oils such as lecithins and fatty acid triglycerides, namely triglycerol esters of saturated and/ or unsaturated, straight or branched carboxylic acid with 8 to 24 carbon atoms, preferably of 12 to 18 carbon atoms whereas the fatty acid triglycerides are preferably chosen from synthetic, half synthetic or natural oils (e.g. cocoglyceride, olive oil, sun flower oil, soybean oil, peanut oil, rape seed oil, sweet almond oil, palm oil, coconut oil, castor oil, hydrogenated castor oil, wheat oil, grape seed oil, macadamia nut oil and others); apolar oils such as linear and/ or branched hydrocarbons and waxes e.g. mineral oils, vaseline (petrolatum); paraffins, squalane and squalene, polyolefins, hydrogenated polyisobutenes and isohexadecanes, favored polyolefins are polydecenes; dialkyl ethers such as dicaprylylether; linear or cyclic silicone oils such as preferably cyclomethicone (octamethylcyclotetrasiloxane; cetyldimethicone, hexamethylcyclotrisiloxane, polydimethylsiloxane, poly(methylphenylsiloxane) and mixtures thereof.

Other fatty components which can advantageously be incorporated in cosmetic or dermatological compositions of the present invention are isoeikosane; neopentylglycoldiheptanoate; propyleneglycoldicaprylate/ dicaprate; caprylic/ capric/ diglycerylsuccinate; butyleneglycol caprylat/ caprat; $C_{12-13}$- alkyllactate; di- $C_{12-13}$- alkyltartrate; triisostearin; dipentaerythrityl hexacaprylat/ hexacaprate; propyleneglycolmonoisostearate; tricaprylin; dimethylisosorbid. Especially beneficial is the use of mixtures $C_{12-15}$- alkylbenzoate and 2- ethylhexylisostearate, mixtures $C_{12-15}$- alkylbenzoate and isotridecylisononanoate as well as mixtures of $C_{12-15}$- alkylbenzoate, 2- ethylhexylisostearate and isotridecylisononanoate.

The oily phase of the compositions of the present invention can also contain natural vegetable or animal waxes such as bee wax, china wax, bumblebee wax and other waxes of insects as well as shea butter and cocoa butter.

<u>Silicone oils</u>

Suitable silicone oils are e.g. such as dimethylpolysiloxane, diethylpolysiloxane, diphenylpolysiloxane, cyclic siloxanes, poly(methylphenylsiloxanes) as well as amino-, fatty acid-, alcohol-, polyether-, epoxy-, fluoro-, glycoside-, and/ or alkyl modified silicone compounds which are liquid or solid at room temperature and mixtures thereof. The number average molecular weight of the dimethicones and poly(methylphenylsiloxanes) is preferably in the range of 100 to 150000 g/ mol. Preferred cyclic siloxanes comprise 4- to 8-membered rings which are for example commercially available as cyclomethicones.

An oil or fatty component is present in an amount of about 1 wt. % to about 50 wt. % of the total weight of the product. The preferred amount of an oil or fatty component is about 2 wt. % to about 25 wt. %, and most preferably about 3 wt. % to about 20 wt. %.

<u>Moisturizing agents</u>

A moisturizing agent may be incorporated into a composition of the present invention to maintain hydration or rehydrate the skin. Moisturizers that prevent water from evaporating from the skin by providing a protective coating are called emollients. Additionally an emollient provides a softening or soothing effect on the skin surface and is generally considered safe for topical use. Preferred emollients include mineral oils, lanolin, petrolatum, capric/ caprylic triglyceraldehydes, cholesterol, silicones such as dimethicone, cyclomethicone, almond oil, jojoba oil, avocado oil, castor oil, sesame oil, sunflower oil, coconut oil and grape seed oil, cocoa butter, olive oil, aloe extracts, fatty acids such as oleic and stearic, fatty alcohols such as cetyl and hexadecyl (ENJAY), diisopropyl adipate, hydroxybenzoate esters, benzoic acid esters of $C_{9-15}$- alcohols, isononyl iso- nonanoate, ethers such as polyoxypropylene butyl ethers and polyoxypropylene cetyl ethers, and $C_{1215}$- alkyl benzoates, and mixtures thereof. The most preferred emollients are hydroxybenzoate esters, aloe vera, $C_{12-15}$- alkyl benzoates, and mixtures thereof. An emollient is present in an amount of about 1 wt. % to about 50 wt. % of the total weight of the product. The preferred amount of emollient is about 2 wt. % to about 25 wt. %, and most preferably about 3 wt. % to about 15 wt. %.

Moisturizers that bind water, thereby retaining it on the skin surface are called humectants. Examples of humectants which can be incorporated into a product of the present invention are glycerin, propylene glycol, polypropylene glycol, polyethylene glycol, lactic acid, sodium lactate, pyrrolidone carboxylic acid, urea, phospholipids, collagen, elastin, ceramides, lecithin, sorbitol, PEG- 4, and mixtures thereof. Additional suitable moisturizers are polymeric moisturizers of the family of water soluble and/ or swellable/ and/ or with water gelating polysaccharides such as hyaluronic acid, chitosan and/ or a fructose rich polysaccharide which is e.g. available as Fucogel®1000 (CAS- Nr. 178463- 23- 5) by SOLABIA S. One or more humectants are optionally present at about 0.5 wt. % to about 8 wt. % in a product of the present invention, preferably about 1 wt. % to about 5 wt. %.

The aqueous phase of the products of the present invention can contain the usual cosmetic additives such as alcohols, especially lower alcohols, preferably ethanol and/ or isopropanol, low diols or polyols and their ethers, preferably propyleneglycol, glycerin, ethyleneglycol, ethyleneglycol monoethyl- or monobutylether, propyleneglycol monomethyl- or -monoethyl- or-monobutylether, diethyleneglycol monomethyl-or monoethylether and analogue products, polymers, foam stabilizers; electrolytes and especially one or more thickeners.

Thickeners

Thickeners that may be used in compositions of the present invention to assist in making the consistency of a product suitable include carbomer, siliciumdioxide, magnesium and/ or aluminum silicates, lipid thickeners, e.g. cetyl alcohol, cetyl palmitate (Cutina CP, Cognis Cooperation), glyceryl myristate (Estol 3650, Uniqema), microcrystalline wax (A&E Connock), myristyl alcohol (Lanette 14, Cognis Cooperation), myristyl lactate (Crodamol ML, Croda Chemicals), beeswax (A&E Connock), stearic acid (Lipo Chemicals), stearyl alcohol (Lanette 18, Cognis Cooperation), polysaccharides and their derivatives such as xanthan gum (Keltrol, CP Kelco), hydroxypropyl cellulose (Klucel, Hercules Incorporated), Hydroxyethylcellulose (Tylose H, Clariant Corporation), polyacrylamides, self emulsifying polyacrylamide, e.g. Salcare SC 91, Salcare SC 96 (Ciba Specialty Chemicals), Sepigel 305 (Seppic), acrylate crosspolymers, preferably a carbomer, such as Carbopole® of type 980, 981, 1382, 2984, 5984, ETD 2001, ETD 2050, Ultrez 10, Ultrez 21 (Noveon Inc.), alone or mixtures thereof. Thickeners can be present in an amount of about 0.01 wt. % to about 8 wt. % in the product of the present invention, preferably, 0.05wt. % to about 5 wt. %.

Neutralizing agents

Examples of neutralizing agents which may be included in the composition of the present invention to neutralize components such as e.g. an emulsifier or a foam builder/ stabilizer include but are not limited to alkali hydroxides such as a sodium and potassium hydroxide; organic bases such as diethanolamine (DEA), triethanolamine (TEA), aminomethyl propanol, and mixtures thereof; amino acids such as arginine and lysine and any combination of any foregoing. The neutralizing agent can be present in an amount of about 0.01 wt. % to about 8 wt. % in the product of the present invention, preferably, 1 wt. % to about 5 wt. %.

Electrolytes

The addition of electrolytes into the product of the present invention may be necessary to change the behavior of a hydrophobic emulsifier. Thus, the emulsions/ microemulsions of this invention may contain preferably electrolytes of one or several salts including anions such as chloride, sulfate, carbonate, borate and aluminate, without being limited thereto. Other suitable electrolytes can be on the basis of organic anions such as, but not limited to, lactate, acetate, benzoate, propionate, tartrate and citrate. As cations preferably ammonium, alkylammonium, alkali- or alkaline earth metals, magnesium-, iron- or zinc-ions are selected. Especially preferred salts are potassium and sodium chloride, magnesium sulfate, zinc sulfate and mixtures thereof. Electrolytes can be present in an amount of about 0.01 wt. % to about 8 wt. % in the product of the present invention.

Film formers

Customary film formers include, for example, chitosan, microcrystalline chitosan, quaternized chitosan, polyvinylpyrrolidone, vinylpyrrolidone/ vinyl acetate copolymers, polymers of quaternary cellulose derivatives containing a high proportion of acrylic acid, collagen, hyaluronic acid and salts thereof and similar compounds.

Polymers

The products according to the invention may comprise additional cosmetically and dermatologically acceptable polymers in order to established the desired properties. For this purpose all anionic, cationic, amphoteric or neutral polymers may be used.

Examples of cationic polymers are Polyquaternium (INCI), e.g. copolymers of vinylpyrrolidone/N- vinylimidazolium salts (Luviquat®FC, Luviquat® HM, Luviquat®MS, Luviquat®Ultracare), copolymers of N- vinylpyrrolidone/ dimethylaminoethylmethacrylate, quaternized with diethylsulfate (Luviquat® PQ 11, INCI: Polyquaternium- 11), copolymers of N- vinylcaprolactam/ N- vinyl- pyrrolidone/ N- vinylimidazolium salts (Luviquat®Hold; INCI: Polyquaternium- 46) ; cationic derivatives of cellulose (Polyquaternium- 4 und- 10), acrylamidocopolymers (Polyquaternium- 7), Chitosan, cationic starch derivatives (INCI: starch hydroxypropyltrimonium chloride, corn starch modified), cationic guar derivates (INCI: hydroxypropyl guar hydroxypropyltrimonium chloride), cationic sun flower seed derivatives (INCI: sun flower seed amidopropyl hydroxyethyldimonium chloride), copolymers of acrylic acid, acrylamide and methacrylamidopropyltrimoniumchlorid (INCI: Polyquaternium- 53), Polyquaternium- 32, Polyquaternium- 28 without being limited thereto. Suitable cationic quaternized polymers are furthermore Merquat® (polymers on the basis of dimethyldiallyl ammoniumchlorid), Gafquat® (quaternary polymers formed by reacting polyvinylpyrrolidone with quaternary ammonium compounds) ; Polymer JR (hydroxyethylcellulose with cationic groups), and cationic polymers on plant basis such as guar polymers, commercially available as Jaguar® grades of Rhodia.

Examples of neutral polymers are polyvinylpyrrolidone, copolymers of N-vinylpyrrolidone and vinylacetate and/or vinylpropionate, polysiloxane, polyvinylcaprolactam and other copolymers of N-vinylpyrrolidone, copolymers of N-vinylpyrrolidone and alkylacrylate or methacrylate monomers with C1-C18 alkyl chains, copolymers of polyvinylalcohol and polyalkylenglycol such as Kollicoats® IR (BASF) or copolymers of other vinyl monomers to polyalkylenglycol, polysiloxane, polyvinylcaprolactam and copolymers with N-Vinylpyrrolidone, polyethylenimine and the salts thereof, polyvinylamine and the salts thereof, cellulose derivatives, chitosan, polyasparaginic acid salts and derivatives thereof, polyethylenimine and the salts thereof, polyvinylamine and the salts thereof such as Luviflex® Swing (partly hydrolysed copolymer of polyvinylacetate and polyethylenglycol by BASF).

Suitable polymers are also non-ionic, water soluble respectively water dispersible polymers or oligomers such as polyvinylcaprolactam, e.g. Luviskol® Plus (BASF), or polyvinylpyrrolidone and copolymers with e.g. vinylesters such as vinylacetate e.g. Luviskol® VA 37 (BASF); polyamide e.g. on the basis of itaconic acid and aliphatic diamines as e.g. described in DE-A-43 33 23.

Additional suitable polymers are nonionic, siloxene-containing, water-soluble or -dispersible polymers, e.g. polyether siloxanes, such as Tegopren® (Goldschmidt) or Belsil® (Wacker).

Preservatives

Examples of preservatives include Methyl-, Ethyl-, Propyl-, Butylparabens, Benzalkonium chloride, 2- Bromo-2- nitro- propane- 1, 3- diol, Dehydroacetic acid, Diazolidinyl Urea, 2- Dichlorobenzyl alcohol, DMDM hydantoin, Formaldehyde solution, Methyldibromoglutaronitrile, Phenoxyethanol, Sodium Hydroxymethylglycinate, Imidazolidinyl Urea, Triclosan and further substance classes listed in the following reference: K. F. De Polo- A short textbook of cosmetology, Chapter 7, Table 7- 2, 7- 3, 7- 4 and 7- 5, p210- 219.

Active ingredients

The cosmetic or dermatological composition of the present invention may further comprise a safe and effective amount of additional skin active agents. The skin active agents included herein are e.g. skin-lightening agents, tanning prevention agents, agents for the treatment of hyperpigmentation, agents for the prevention or reduction of acne, vitamins, emollients, non-steroidal anti-inflammatory agent, topical anaesthetics, antiseptics, antimicrobial (e.g. bacteria-inhibiting) and anti-fungal actives, skin soothing agents, skin barrier repair agents, anti-wrinkle agents, anti-skin atrophy actives, lipids, plant extracts and plant derived actives, sebum inhibitors, skin sensates, protease inhibitors, skin thightening agents, skin anti-cellulites agents, anti-itch agents, hair grow inhibitors, desquamation enzyme enhancers, anti-glycation agents, chelators and sequestrants, opacifiers, radical scavengers, desquamatory actives, anti-acne actives, anti-oxidants, and mixtures thereof.

When included, the present cosmetic or dermatological composition comprise at least 0.001 wt. % of the additional skin active ingredient. Generally, an amount of about 0.001 wt. % to about 30 wt. %, preferably from about 0.001 wt. % to about 10 wt. %, most preferably in an amount of 0.01 to 5 wt. % of an additional skin active agent is used.

The type and amount of the skin active agents are selected so that the inclusion of a specific agent does not affect the stability of the cosmetic or dermatological composition.

Additional skin- lightening, anti aging or anti- acne actives can be added to the cosmetic or dermatological, compositions of the invention if an additional increase of the efficacy is desired. For example, the use of combinations of skin lightening agents may be advantageous in that they may provide skin lightening benefit through different mechanisms. Preferably, the additional skin lightening agents is selected from bis- pantoyl- cystamine, arbutin and alpha-arbutin, deoxyarbutin, undecylenoyl phenyl alanine (for example, SEPIWHITE MSH available from Seppic), octadecenedioic acid (for example ARLATONE DIOIC DCA available from Uniquema), oenothera biennis sead extract, and pyrus malus (apple) fruit extract, Melfade (available from Pentapharm), MELAWHITE (available from Pentapharm), Melanostatine DM (available from Laboratories Seporga), FADEOUT (available from Pentapharm), GATULINE WHITENING (available from Gattefosse), kojic acid, kojic dipalmitate, lactic acid, azelaic acid, gallic acid and its derivatives, hydroquinone, mulberry extract, lemon extract, phyllanthus emblica fruit extract (available as Emblica™), leucocyte extract, bearberry extract, licorice extract and mixtures thereof. Examples of additional, other skin lightening agents, which may be present in the compositions of the present invention are especially those disclosed in WO 2004/062635, WO 2004/037213, and DE 102 38 449.

Preferable additional skin lightening agents which may be used in the cosmetic or dermatological compositions according to the present invention are

Kojic acid or derivatives thereof, which may be present in the compositions of the present invention in an amount from about 0.05 wt.-% to about 5 wt.-%;

Arbutin or derivatives thereof which may be present in compositions of the present invention in an amount from about 0.05 wt.-% to about 5 wt.-%;

Hydroquinone or derivatives thereof which may be present in the compositions of the present invention in an amount from about 0.05 wt.-% to about 2 wt.-%;

Phyllanthus Emblica fruit extract (trade name: Emblica™), which may be present in the compositions of the present invention in an amount from 0.05 wt.- % to about 3 wt.- %;

Leucocyte extract, which may be present in the compositions of the present invention in an amount from 0.05 wt.-% to about 3 wt.-%;

Bearberry extract, which may be present in the compositions of the present invention in an amount from 0.05 wt.% to about 3 wt.-%;

Licorice extract, which may be present in the skin care compositions of the present invention in an amount from 0.05 wt.% to about 3 wt.-%; and

Mulberry extract, which may be present in the skin care compositions of the present invention in an amount from 0.05 wt.% to about 3 wt.%.

Preferable additional anti-acne agents, which may be used in the cosmetic or dermatological compositions according to the present invention are salicylic acid and its salts or derivatives and other alpha and beta-hydroxy acids, salts or derivatives, triclosan, plant extracts, glycyric acid or salt, clindamycin, benzoyl peroxide, resorcinol.

Examples of active ingredients are e.g. glycerol, urea, guanidine (e.g., amino guanidine) ; vitamins and derivatives thereof such as vitamin A (e.g., retinoid derivatives such as retinol, retinal, retinyl palmitate or retinyl propionate), vitamin E (e.g., tocopherol acetate), and vitamin $B_5$ (e.g., panthenol), vitamin $B_6$, vitamin $B_{12}$, vitamin K, vitamin D, Coenzyme Q10 (e.g. ALL- Q® plus) and folic acid and mixtures thereof, wax- based synthetic peptides (e.g., octyl palmitate and tribehenin and sorbitan isostearate and palmitoyl- oligopeptide), amino acids, oligopeptides, and bioactive peptides (e.g., Matrixyl™ [pentapeptide derivative or BeauActive MTP), anti- acne medicaments; antioxidants (e.g., phytosterols, lipoic acid) ; flavonoids or polyphenols (e.g., isoflavones, phytoestrogens) ; skin soothing and healing agents such as aloe vera extract, allantoin; agents suitable for aesthetic purposes such as essential oils or aromatic compounds (e.g., clove oil, menthol, camphor, eucalyptus oil, and eugenol), hydroxy acids, farnesol, antifungal actives such as bisabolol, alkyldiols such as 1, 2- pentanediol, hexanediol or 1, 2- octanediol, panthenol, phytol, phytantriol, ceramides and pseudoceramides, protein hydrolysates, AHA acids, polyunsaturated fatty acids, plant extracts like kinetin, DNA or RNA and their fragmentation products or carbohydrates, conjugated fatty acids, carnitin, carnosine, biochinonen, phytofluen, phytoen, and their corresponding derivatives, carotinoid derivatives such as beta carotene, lycopene or asthaxanthene.

A vitamin E derivative for use in the present invention is tocopheryl acetate. Tocopheryl acetate may be present in the skin care products in an amount from about 0.05 wt.-% to about 5 wt.%. Another vitamine E derivative of interest is tocopheryl linoleate. Tocopheryl linoleate may be present in the skin care composition in an amount from about 0.05 wt.-% to about 5 wt.-%. Also the vitamin E alcohol can be added as additional active, preferentially as mixed tocopherol in an amount from about 0.01 wt-% to 1.00 wt.-%

Examples of vitamins from the B complex for use in the present invention are vitamin $B_6$. Vitamin $B_6$ may be present in the skin care products in an amount from about 0.01 wt-% to about 1.00 wt.-%.

Panthenol may be present in the skin care products in an amount from about 0.05 wt.% to about 5.00 wt.%. Phytantriol may be present in the skin care products in an amount from about 0.01 wt.% to about 5.0 wt.%. Bisabolol may be present in the skin care products in an amount from about 0.05 wt.-% to about 5.00 wt.-%.

Regarding the kind of the topical cosmetic and pharmaceutical composition and the preparation of the topical cosmetic and dermatological preparations as well as for further suitable additives, it can be referred to the pertinent literature, e.g. to Novak G.A., Die kosmetischen Präparate - Band 2, Die kosmetischen Präparate - Rezeptur, Rohstoffe, wissenschaftliche Grundlagen (Verlag für Chem. Industrie H. Ziolkowski KG, Augsburg).

In another embodiment the invention relates to cosmetic or dermatological compositions with the definitions and preferences as given above comprising about 3 wt.-% of an ascorbyl phosphate.

In a further embodiment the invention relates to the use of an ascorbyl phosphate and niacinamide to solubilize biotin in water. Furthermore the invention relates a process for the preparation of cosmetic or dermatological compositions, the process comprising the solubilization of biotin in a mixture of water, an ascorbyl phosphate and niacinamide. Preferably the amount of ascorbyl phosphate as well as the amount of niacinamide used for the solubilization of biotin is higher than the amount of biotin used. Advantageously, the amount of ascorbyl phosphate as well as the amount of niacinamide is at least 1.5 times the amount of biotin used. Preferably the amount of niacinamide used for the solubilization of biotin is equal or higher than the amount of ascorbyl phosphate used. In particular, the ratio of niacinamide to the ascorbyl phosphate is selected in the range of 1:1 to 1:100, preferably in the range of 1:1 to 1:50, in particular in the range of 1:1 to 1:5 such as in a ratio of 1:3.

In another embodiment the present invention relates to the use of an ascorbyl phosphate in combination with niacinamide for suppressing the crystallization of biotin in cosmetic or dermatological compositions. Preferably the amount of ascorbyl phosphate as well as the amount of niacinamide for suppressing the crystallization of biotin in cosmetic or dermatological compositions is higher than the amount of biotin used. Advantageously, the amount of ascorbyl phosphate as well as the amount of niacinamide is at least 1.5 times the amount of biotin. Preferably the amount of niacinamide used for suppressing the crystallization of biotin is equal or higher than the amount of ascorbyl phosphate used. In particular, the ratio of niacinamide to the ascorbyl phosphate is selected in the range of 1:1 to 1:100, preferably in the range of 1:1 to 1:50, in particular in the range of 1:1 to 1:5 such as in a ratio of 1:3.

The invention is illustrated further by the Examples without being limited thereto.

**Example 1: Solubility of Biotin**

The solubility of Biotin was assessed in aqueous niacinamide (Niacinamide PC, DSM Nutritional Products) / sodium ascorbyl phosphate (STAY-C® 50, DSM Nutritional Products) solutions in different concentration. As reference, the solubility of biotin in water was assessed.

After the addition of 5 g of water into a 10 ml glass vial, STAY-C® 50 and Niacinamide PC were added in order to yield the ratios and wt. % as indicated in table 1. The mixtures were stirred with a magnetic stirrer at RT until STAY-C® 50 and Niacinamide PC were completely dissolved. Then, biotin was added in about 1 mg steps to the mixture until the solution becomes turbid. This experiment was repeated twice.

The sum of the amount of biotin which was added before the turbidity occurred is taken as the solubility of Biotin. The result was calculated with:

$$\text{Solubility of Biotin in wt. \%} = \frac{\text{Biotin solubilized [mg]} \times 100}{\text{weight of water [mg]}}$$

**Table 1**

| STAY-C® 50 [wt. %] | Niacinamide PC [wt. %] | Ratio | Solubility of Biotin [wt. %] |
|---|---|---|---|
| -- | -- | Reference | 0.022 |
| 0.1 | 1 | 1:10 | 0.15 |
| 0.1 | 3 | 1:30 | 0.17 |
| 0.1 | 5 | 1:50 | 0.20 |
| 0.1 | 10 | 1:100 | 0.26 |
| 0.5 | 1 | 1:2 | 0.33 |
| 0.5 | 3 | 1:6 | 0.38 |
| 0.5 | 5 | 1:10 | 0.41 |
| 0.5 | 10 | 1:20 | 0.47 |
| 1.0 | 1 | 1:1 | 0.64 |
| 1.0 | 3 | 1:3 | 0.66 |

(continued)

| STAY-C® 50 [wt. %] | Niacinamide PC [wt. %] | Ratio | Solubility of Biotin [wt. %] |
|---|---|---|---|
| 1.0 | 5 | 1:5 | 0.70 |
| 1.0 | 10 | 1:10 | 0.76 |
| 3.0 | 1 | 3:1 | 1.53 |
| 3.0 | 3 | 1:1 | 1.65 |
| 3.0 | 5 | 1:1.6 | 1.71 |
| 3.0 | 10 | 1:3.3 | 1.78 |

As can be seen from the results presented in table 1 the solubility of biotin can be significantly enhanced by the addition of STAY-C® 50 and Niacinamide PC.

**Example 2: Moisturizing night anti-age spot Cream (W/O)**

| Phase | Ingredients | INCI Name | % w / w |
|---|---|---|---|
| A | Cremophor WO-7 | PEG-7 Hydrogenated Castor Oil | 6.00 |
| | Elfacos ST-9 | PEG-45/Dodecyl Glycol Copolymer | 2.00 |
| | Paraffin Oil | Mineral Oil | 10.00 |
| | Jojoba Oil | Simmondsia Chinensis (Jojoba) Seed Oil | 5.00 |
| | Myritol 318 | Caprylic/Capric Triglyceride | 5.00 |
| | Paracera M | Microcrystalline Wax | 2.00 |
| | Phenonip | Phenoxyethanol & Methylparaben & Ethylparaben & Butylparaben & Propylparaben & Isobutylparaben | 0.80 |
| B | Edeta BD | Disodium EDTA | 0.10 |
| | 1,2-Propanediol | Propylene Glycol | 5.0 |
| | Water dem. | Aqua | Ad 100 |
| | Panthenol 75L | Panthenol | 0.50 |
| | **STAY-C® 50** | Sodium Ascorbyl Phosphate | 2.00 |
| | **Niacinamide** | Niacinamide | 5.00 |
| | **Biotin** | Biotin | 1.00 |

**Procedure**

1 Heat part A up to 85°C; and heat also part B up to 35°C.
2 When both have the temperature, add part A to part B while homogenizing intensively. Cool down the product at ambient temperature, while stirring. It is generally recommended to use vacuum while producing the emulsion.

**Example 3: Anti-Wrinkle Cream**

| Phase | Ingredients | INCI Name | % w / w |
|---|---|---|---|
| A | Estol 3650 | Glyceryl Myristate | 2.50 |
| | Lanette 16 | Cetyl Alcohol | 2.50 |

(continued)

| Phase | Ingredients | INCI Name | % w / w |
|---|---|---|---|
| | PARSOL® MCX | Ethylhexyl Methoxycinnamate (Octinoxate; USAN) | 5.00 |
| | PARSOL® 1789 | Butyl Methoxydibenzoylmethane (Avobenzone; USAN) | 2.00 |
| | PARSOL® 340 | Octocrylene (Octocrilene; USAN) | 1.70 |
| | Macadamianussöl raffiniert | Macadamia Ternifolia Seed Oil | 2.00 |
| | Finsolv TN | C12-15 Alkyl Benzoate | 4.00 |
| | Butylated Hydroxytoluene | BHT | 0.05 |
| | Dow Corning 200/350 cs | Dimethicone | 0.50 |
| | Phenonip | Phenoxyethanol & Methylparaben & Ethylparaben & Butylparaben & Propylparaben & Isobutylparaben | 0.80 |
| | Brij 72 | Steareth-2 | 2.00 |
| | Brij 721 | Steareth-21 | 2.00 |
| B | Glycerin | Glycerin | 3.00 |
| | Edeta BD | Disodium EDTA | 0.10 |
| | Keltrol CG-T | Xanthan Gum | 0.20 |
| | Carbopol ETD 2001 | Carbomer | 0.25 |
| | Water dem. | Aqua | Ad 100 |
| C | Water dem. | Aqua | 15.0 |
| | Natriumdisulfit | Sodium Metabisulfite | 0.05 |
| | **STAY-C® 50** | Sodium Ascorbyl Phosphate | 0.5 |
| | **Niacinamide PC** | Niacinamide | **1.50** |
| | **Biotin** | Biotin | **0.20** |
| D | ALL-Q® plus | Ubiquinone & Tocopheryl Acetate & C12-15 Alkyl Benzoate | 1.00 |

**Procedure**

1 Heat part A up to 85°C; and heat also part B up to 85°C.
2 When both have the same temperature, add part B to part A while homogenizing intensively. Cool down the product to 35°C while stirring.
3 Solubilize Biotin in C and add part C and D homogenize intensively again. It is generally recommended to use vacuum while producing the emulsion.

**Example 4: Anti-aging cream**

| Phase | Ingredients | INCI Name | % w / w |
|---|---|---|---|
| A | AMPHISOL® A | Cetyl Phosphate | 2.00 |
| | Estol 3650 | Glyceryl Myristate | 4.00 |
| | Lanette 16 | Cetyl Alcohol | 1.00 |
| | Dow Corning 200/350 cs | Dimethicone | 2.00 |
| | Sweet Almond Oil | Prunus Amygdalus Dulcis (Sweet Almond) Oil | 3.00 |
| | Ceraphyl 375 | Isostearyl Neopentanoate | 7.00 |
| | dl-alpha -Tocopheryl Acetate | Tocopheryl Acetate | 2.00 |
| | Phenonip | Phenoxyethanol & Methylparaben & Ethylparaben & Butylparaben & Propylparaben & Isobutylparaben | 0.80 |

(continued)

| Phase | Ingredients | INCI Name | % w / w |
|---|---|---|---|
| B | Glycerin | Glycerin | 5.00 |
| | Triethanolamine (T.E.A.) | Triethanolamine | 0.90 |
| | Water dem. | Aqua | Ad 100 |
| C | Sepigel 305 | Polyacrylamide & C13-14 Isoparaffin & Laureth-7 | 3.00 |
| D | Water dem. | Aqua | 20.0 |
| | **STAY-C® 50** | Sodium Ascorbyl Phosphate | **0.50** |
| | **Niacinamide PC** | Niacinamide | **2.00** |
| | **Biotin** | | **0.35** |

**Procedure**

1 Heat part A to 85°C while stirring.
2 Heat part B to 80°C and add to part A while stirring and homogenizing the emulsion.
3 Cool down the emulsion to 55°C and add part C.
4 Cool down further to 35°C and add part D
It is highly recommended to remove oxygen as much as possible by using vacuum while producing the product.

**Example 5: Regenerating Cream**

| Phase | Ingredients | INCI Name | % w / w |
|---|---|---|---|
| A | Estol 3650 | Glyceryl Myristate | 1.50 |
| | Lanette 16 | Cetyl Alcohol | 1.50 |
| | Finsolv TN | C12-15 Alkyl Benzoate | 4.00 |
| | Phenonip | Phenoxyethanol & Methylparaben & Ethylparaben & Butylparaben & Propylparaben & Isobutylparaben | 0.80 |
| | Lanol 99 | Isononyl Isononanoate | 2.00 |
| | Brij 72 | Steareth-2 | 1.50 |
| | Brij 721 | Steareth-21 | 1.50 |
| B | 1,3-Butylenglykol | Butylene Glycol | 2.00 |
| | Glycerin | Glycerin | 3.00 |
| | Edeta BD | Disodium EDTA | 0.10 |
| | Keltrol CG-T | Xanthan Gum | 0.30 |
| | Carbopol Ultrez 21 | Acrylates/C10-30 Alkyl Acrylate Crosspolymer | 0.25 |
| | Water dem. | Aqua | Ad 100 |
| C | Water dem. | Aqua | 20.0 |
| | **STAY-C® 50** | Sodium Ascorbyl Phosphate | **1.00** |
| | Natriumdisulfit | Sodium Metabisulfite | 0.05 |
| | **Niacinamide** | Niacinamide | **5.00** |
| | **Biotin** | Biotin | **0.30** |
| D | Citric Acid, Anhydrous | Citric Acid | 0.50 |
| E | Vitamin A Palmitate | Retinyl Palmitate | 0.30 |

**Procedure**

1 Heat part A up to 85°C; and heat also part B up to 85°C.
2 When both have the same temperatures add part B to part A while homogenizing intensively. Cool down the product to 35°C while stirring.
3 Now add part C under stirring. Then add part D and E and homogenize intensively again.
4 It is generally recommended to use vacuum while producing the emulsion.

**Example 6: Skin lightening cream**

| Phase | Ingredients | INCI Name | % w / w |
|---|---|---|---|
| A | Estol 3650 | Glyceryl Myristate | 2.50 |
| | Lanette 16 | Cetyl Alcohol | 2.50 |
| | PARSOL® EHS | Ethylhexyl Salicylate (Octisalate; USAN) | 5.00 |
| | PARSOL® 1789 | Butyl Methoxydibenzoylmethane (Avobenzone; USAN) | 2.00 |
| | PARSOL® 340 | Octocrylene (Octocrilene; USAN) | 1.70 |
| | Sweet Almond Oil | Prunus Amygdalus Dulcis (Sweet Almond) Oil | 2.00 |
| | Finsolv TN | C12-15 Alkyl Benzoate | 8.00 |
| | Butylated Hydroxytoluene | BHT | 0.05 |
| | Dow Corning 200/350 cs | Dimethicone | 0.50 |
| | dl-alpha-Tocopheryl Acetate | Tocopheryl Acetate | 1.00 |
| | Phenonip | Phenoxyethanol & Methylparaben & Ethylparaben & Butylparaben & Propylparaben & Isobutylparaben | 0.80 |
| | Brij 72 | Steareth-2 | 2.00 |
| | Brij 721 | Steareth-21 | 2.00 |
| | Dow Corning 345 Fluid | Cyclopentasiloxane & Cyclohexasiloxane | 4.00 |
| B | Water dem. | Aqua | Ad 100 |
| | 1,3-Butylenglykol | Butylene Glycol | 2.00 |
| | Glycerin | Glycerin | 3.00 |
| | Edeta BD | Disodium EDTA | 0.10 |
| | Keltrol CG-T | Xanthan Gum | 0.20 |
| | Carbopol Ultrez 21 | Acrylates/C10-30 Alkyl Acrylate Crosspolymer | 0.25 |
| C | Water dem. | Aqua | 20.00 |
| | **STAY-C® 50** | Sodium Ascorbyl Phosphate | **1.00** |
| | **Niacinamide PC** | Niacinamide | **2.00** |
| | Natriumdisulfit | Sodium Metabisulfite | 0.05 |
| | **Biotin** | Biotin | **0.35** |

**Procedure**

1 Heat part A up to 85°C; and heat also part B up to 85°C.
2 When both have the same temperature, add part B to part A while homogenizing intensively. Cool down the product to 35°C while stirring.
3 Now add part C and homogenizes intensively again.
It is generally recommended to use vacuum while producing the emulsion.

**Example 7: Anti-acne Gel**

| Phase | Ingredients | INCI Name | % w/w |
|---|---|---|---|
| A | Natrosol 250 HHR | Hydroxyethylcellulose | 1.20 |
| B | Natriumdisulfit | Sodium Metabisulfite | 0.10 |
| | **STAY-C® 50** | Sodium Ascorbyl Phosphate | **2.00** |
| | D-Panthenol 75 L | Panthenol | 1.15 |
| | Water dem. | Aqua | Ad 100 |
| | **Niacinamide PC** | Niacinamide | **7.50** |
| | **D-Biotin** | Biotin | **1.00** |
| C | 1,2-Propanediol | Propylene Glycol | 5.00 |
| | Ethanol | Alcohol | 3.00 |
| | Abil B 88183 | PEG/PPG-20/6 Dimethicone | 0.50 |
| D | Ethanol | Alcohol | 7.00 |
| | Cremophor RH 410 | PEG-40 Hydrogenated Castor Oil | 0.80 |
| | Perfume | Perfume | 0.40 |
| E | Citric Acid, Anhydrous | Citric Acid | 1.00 |

**Procedure**

1 Add Natrosol HHR to part B while mixing intensively. When everything is homogeneous wait for a while, until a clear gel is obtained.
2 Then add part C and D carefully under stirring. At the end add part E

## Claims

**1.** A cosmetic or dermatological composition comprising

(a) 0.1 to 5 wt. % of an ascorbyl phosphate;
(b) 0.5 to 10 wt. % of niacinamide
(c) 0.005 to 3 wt.-% biotin or a salt thereof and
(d) a cosmetically acceptable carrier.

**2.** The cosmetic or dermatological composition according to claim 1, which is a topical composition.

**3.** The cosmetic or dermatological composition according to claim 1 or 2, which furthermore comprises from 20 to 99 wt.-% of water.

**4.** The composition according to anyone of claims 1 to 3, wherein the amount of biotin or a salt thereof is in the range of 0.01 to 1 wt.-%.

**5.** The composition according to anyone of claims 1 to 4, wherein the biotin or a salt thereof is D- (+)- Biotin or a salt thereof.

**6.** The composition according to anyone of claims 1 to 5, wherein the ascorbyl phosphate is sodium or sodium magnesium or sodium calcium ascorbyl phosphate or mixtures thereof.

7. The composition according to anyone of claims 1 to 6, wherein the ascorbyl phosphate is trisodium L- ascorbyl- 2-monophosphate.

8. The composition according to anyone of claims 1 to 7, wherein the amount of the ascorbyl phosphate and the amount of niacinamide is at least 1.5 times the amount of biotin or a salt thereof.

9. Use of a composition according to any one of claims 1 to 8 as moisturizing or skin-lightening agent.

10. A composition according to any one of claims 1 to 8 for use as anti-ageing or anti-acne agent.

11. Use of a composition according to any one of claims 1 to 8 for the prevention, reduction or treatment of wrinkles.

12. Use of ascorbyl phosphate and niacinamide for suppressing the crystallization of biotin in cosmetic or dermatological compositions.

13. The use according to claim 12, wherein the amount of the ascorbyl phosphate and the amount of niacinamide is at least 1.5 times the amount of biotin.

14. A process for the preparation of cosmetic or dermatological compositions, the process comprising the solubilization of biotin in a mixture of water, an ascorbyl phosphate and niacinamide.

**Patentansprüche**

1. Kosmetische oder dermatologische Zusammensetzung, die Folgendes umfasst:

   (a) 0,1 bis 5 Gew.-% eines Ascorbylphosphats;
   (b) 0,5 bis 10 Gew.-% Niacinamid
   (c) 0,005 bis 3 Gew.-% Biotin oder ein Salz davon, und
   (d) einen kosmetisch unbedenklichen Träger.

2. Kosmetische oder dermatologische Zusammensetzung nach Anspruch 1, bei der es sich um eine topische Zusammensetzung handelt.

3. Kosmetische oder dermatologische Zusammensetzung nach Anspruch 1 oder 2, die weiterhin 20 bis 99 Gew.-% Wasser umfasst.

4. Zusammensetzung nach einem der Ansprüche 1 bis 3, wobei die Menge an Biotin oder eines Salzes davon im Bereich von 0,01 bis 1 Gew.-% liegt.

5. Zusammensetzung nach einem der Ansprüche 1 bis 4, wobei es sich bei dem Biotin oder einem Salz davon um D-(+)- Biotin oder ein Salz davon handelt.

6. Zusammensetzung nach einem der Ansprüche 1 bis 5, wobei es sich bei dem Ascorbylphosphat um Natrium- oder Natriummagnesium- oder Natriumcalciumascorbylphosphat oder Mischungen davon handelt.

7. Zusammensetzung nach einem der Ansprüche 1 bis 6, wobei es sich bei dem Ascorbylphosphat um Trinatrium- L-ascorbyl- 2- monophosphat handelt.

8. Zusammensetzung nach einem der Ansprüche 1 bis 7, wobei die Ascorbylphosphatmenge und die Niacinamidmenge mindestens 1,5-mal der Menge an Biotin oder einem Salz davon entsprechen.

9. Verwendung einer Zusammensetzung nach einem der Ansprüche 1 bis 8 als feuchtigkeitsspendendes oder hautaufhellendes Mittel.

10. Zusammensetzung nach einem der Ansprüche 1 bis 8, zur Verwendung als Mittel gegen das Altern oder gegen Akne.

11. Verwendung einer Zusammensetzung nach einem der Ansprüche 1 bis 8 zur Vorbeugung, Reduktion oder Behandlung von Falten.

**12.** Verwendung von Ascorbylphosphat und Niacinamid zum Unterdrücken des Auskristallisierens von Biotin in kosmetischen oder dermatologischen Zusammensetzungen.

**13.** Verwendung nach Anspruch 12, wobei die Ascorbylphosphatmenge und die Niacinamidmenge mindestens 1,5-mal der Menge an Biotin entsprechen.

**14.** Verfahren zur Herstellung von kosmetischen oder dermatologischen Zusammensetzungen, wobei bei dem Verfahren Biotin in einer Mischung von Wasser, einem Ascorbylphosphat und Niacinamid solubilisiert wird.

**Revendications**

**1.** Composition cosmétique ou dermatologique, comprenant

(a) de 0,1 à 5% en poids d'un phosphate d'ascorbyle ;
(b) de 0,5 à 10% en poids de niacinamide ;
(c) de 0,005 à 3% en poids de biotine ou d'un sel de celle-ci ; et
(d) un véhicule pharmaceutiquement acceptable.

**2.** Composition cosmétique ou dermatologique selon la revendication 1, qui est une composition topique.

**3.** Composition cosmétique ou dermatologique selon la revendication 1 ou 2, comprenant en outre de 20 à 99% en poids d'eau.

**4.** Composition selon l'une quelconque des revendications 1 à 3, dans laquelle la quantité de biotine ou d'un sel de celle-ci se trouve dans la plage allant de 0,01 à 1% en poids.

**5.** Composition selon l'une quelconque des revendications 1 à 4, dans laquelle la biotine ou un sel de celle- ci est la D- (+)- biotine ou un sel de celle- ci.

**6.** Composition selon l'une quelconque des revendications 1 à 5, dans laquelle le phosphate d'ascorbyle est le phosphate d'ascorbyle de sodium ou de sodium et de magnésium ou de sodium et de calcium, ou des mélanges de ceux-ci.

**7.** Composition selon l'une quelconque des revendications 1 à 6, dans laquelle le phosphate d'ascorbyle est le L-ascorbyl- 2- monophosphate trisodique.

**8.** Composition selon l'une quelconque des revendications 1 à 7, dans laquelle la quantité de phosphate d'ascorbyle et la quantité de niacinamide est au moins 1,5 fois celle de la biotine ou d'un sel de celle-ci.

**9.** Utilisation d'une composition selon l'une quelconque des revendications 1 à 8, comme agent hydratant ou éclaircissant de la peau.

**10.** Composition selon l'une quelconque des revendications 1 à 8, pour une utilisation comme agent anti-âge ou anti-acnéique.

**11.** Utilisation d'une composition selon l'une quelconque des revendications 1 à 8, pour la prévention, la réduction ou le traitement des rides.

**12.** Utilisation de phosphate d'ascorbyle et de niacinamide pour la suppression de la cristallisation de la biotine dans des compositions cosmétiques ou dermatologiques.

**13.** Utilisation selon la revendication 12, dans laquelle la quantité de phosphate d'ascorbyle et la quantité de niacinamide est au moins 1,5 fois celle de la biotine.

**14.** Procédé de préparation de compositions cosmétiques ou dermatologiques, le procédé comprenant la solubilisation de biotine dans un mélange d'eau, d'un phosphate d'ascorbyle et de niacinamide.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0895776 A
- EP 0358584 B1
- EP 0538431 B1
- EP 0709080 A1
- EP 1471995 A
- EP 1046391 A
- WO 2005080341 A1
- EP 0514491 B1
- EP 0780119 A1
- US 5605680 A
- DE 433323 A
- WO 2004062635 A
- WO 2004037213 A
- DE 10238449


### Non-patent literature cited in the description


- **K. F. DE POLO.** A short textbook of cosmetology. 210-219
- Novak G.A., Die kosmetischen Präparate. **H. ZIOLKOWSKI.** Die kosmetischen Präparate - Rezeptur, Rohstoffe, wissenschaftliche Grundlagen. Verlag für Chem. Industrie, vol. 2